# EUROPÄISCHE PATENTSCHRIFT

(11) **EP 1 095 927 B1**
(45) Veröffentlichungstag und Bekanntmachung des Hinweises auf die Patenterteilung: **02.04.2003**
(21) Anmeldenummer: 00122595.2
(22) Anmeldetag: 17.10.2000
(51) Int. Cl.: C07C 39/04, C07C 37/86

(54) **Verfahren zur Entfernung von Verunreinigungen aus Phenol mittels zumindest eines sauren Katalysators**
Process for the removal of impurities from phenol by treatment with at least one acid catalyst
Procédé pour l'élimination d'impuretés du phénol par traitement avec au moins un catalyseur acide

(30) Priorität: 26.10.1999 DE 19951373
(43) Veröffentlichungstag der Anmeldung: 02.05.2001
(73) Patentinhaber: INEOS Phenol GmbH & Co. KG, 45966 Gladbeck (DE)
(72) Erfinder: Van Barneveld, Heinrich, Dr., 46244 Bottrop (DE); Jordan, Wilfried, Dr., 46282 Dorsten (DE); Schnurr, Otto, Dr., 2950 Kapellen (BE); Gerlich, Otto, Dr., 45966 Gladbeck (DE); Liefooghe, Hugo H.J.M., Dr., 2650 Edegem (BE)
(74) Vertreter: Sternagel, Fleischer, Godemeyer & Partner

(56) Entgegenhaltungen:
- EP-A- 1 018 501
- FR-A- 1 302 848

## Beschreibung

Die vorliegende Erfindung betrifft ein Verfahren zur Entfernung von Verunreinigungen aus Phenol mittels zumindest eines sauren Katalysators.

Die Herstellung von Phenol durch Spalten von Cumolhydroperoxid ist grundsätzlich bekannt. Das bei der Spaltung erhaltene Reaktionsgemisch wird im allgemeinen im Anschluß an eine Neutralisation einer fraktionierten Destillation unterworfen. Bei dieser Destillation wird zunächst Aceton als erstes Hauptprodukt abdestilliert. Danach werden bei Fortsetzung der Destillation noch vorhandenes Isopropylbenzol, alpha-Methylstyrol und Wasser als weitere Fraktionen erhalten.

Als zweites Hauptprodukt wird danach Rohphenol über Kopf abdestilliert, wobei im Sumpf dieser Kolonne noch ein Gemisch aus Phenol, Acetophenon, Dimethylphenylcarbinol und p-Cumylphenol und eine Reihe von Kondensations- und Polymerisationsprodukten verbleiben.

Dieses Rohphenol enthält noch Verunreinigungen, welche die Weiterverarbeitung des Phenols zu Folgeprodukten erschweren oder empfindlich stören. Bei diesen Verunreinigungen handelt es sich insbesondere um Ketone, wie z.B. Mesityloxid, Isomesityloxid, Methylisobutylketon, Hydroxyaceton, Acetophenon, sowie um ungesättigte Verbindungen und/oder Methylbenzofuran. Diese Verbindungen lassen sich destillativ nicht vollständig aus dem Phenol entfernen.

In DE 1 668 952 wird ein Verfahren zur Gewinnung von Reinphenolen, die nahezu frei von Mesityloxid sind, aus Rohphenolen, die bei der Spaltung von Hydroperoxiden alkylaromatischer Kohlenwasserstoffe in Anwesenheit von Säuren und nachfolgender Fraktionierung des Reaktionsgemisches erhalten wurden, durch Leiten über Festbettkatalysatoren aus makroretikularen Ionenaustauschern, die aktive Sulfonsäuregruppen oder ähnliche aktive saure Gruppen enthalten, bei Temperaturen zwischen 45 und 200 °C und anschließende Destillation, beschrieben. Bei diesem Verfahren erfolgt die Behandlung der flüssigen oder dampfförmigen Rohphenole ohne Beimischung von Wasser. Gegebenenfalls wird das Reaktionsgemisch vor Durchführung der Destillation neutralisiert. Mit Hilfe dieses Verfahrens lassen sich schwach reaktive Carbonylverbindungen wie z.B. Methylisobutylketon und Methylcyclopentenon sowie Verbindungen wie z.B. 2-Methylbenzofuran gar nicht oder nur unvollständig aus dem Rohphenol entfernen. Ursache dafür ist vermutlich eine Gleichgewichtseinstellung der Edukte mit den Reaktionsprodukten beziehungsweise das Verhindern der Weiterreaktion der Reaktionsprodukte durch gebildetes Reaktionswasser.

Aus FR-A-1 302 848 ist außerdem bekannt, daß die Entfernung von Schwefelsäure aus dem bei der säurekatalysierten Spaltung von Cumolhydroperoxid entstehenden Spaltprodukt ohne Extraktion durch den Einsatz von Anionenaustauschern möglich ist.

US 5 414 154 lehrt ein Verfahren zur Verringerung von Methylbenzofuran Verunreinigungen in Phenol, welches aus der Cumolhydroperoxid Spaltung erhalten wurde. Bei diesem Verfahren wird das Rohphenol so behandelt, daß es einen Acetol-Gehalt von kleiner 260 ppm aufweist. Das so behandelte Rohphenol wird in Kontakt zu einem sauren Katalysator gebracht, wodurch Methylbenzofuran zu höher siedenden Verbindungen umgesetzt wird, welche aus dem Rohphenol durch Destillation entfernt werden. Die Durchführung dieses Verfahrens ist auf den Einsatz von Rohphenol beschränkt, welches einen Anteil von Acetol kleiner 260 ppm aufweist.

EP-A-1 018 501 (Veröffentlichungstag: 12.07.2000) beschreibt ein Verfahren zur Entfernung von organischen und/oder anorganischen Säuren aus Spaltproduktphasen.

Aufgabe der vorliegenden Erfindung war es deshalb ein Verfahren zur Entfernung von Verunreinigungen aus Phenol bereitzustellen, welches, weitgehend unabhängig von der Zusammensetzung der Verunreinigungen, in der Lage ist, Phenol auf einfache und möglichst wenig energieintensive Weise von Verunreinigungen abzutrennen.

Überraschenderweise wurde gefunden, daß ein Verfahren zur Entfernung von Verunreinigungen aus Phenol mittels zumindest eines sauren Katalysators, welches dadurch gekennzeichnet ist, daß das zu reinigende Phenol nach einer ersten Behandlung mit einem sauren Katalysator destillativ behandelt wird und die bei der Destillation erhaltene Phenolphase zumindest ein weiteres Mal mit einem sauren Katalysator behandelt wird, es ermöglicht, reaktive Verunreinigungen aus Phenol, wobei die Zusammensetzung der Verunreinigungen kaum Einfluß auf die Wirksamkeit des Verfahrens haben, auf einfache und wenig energieintensive Weise zu entfernen.

Gegenstand der vorliegenden Erfindung ist deshalb ein Verfahren zur Entfernung von Verunreinigungen aus Phenol durch Umwandeln dieser Verunreinigungen mittels zumindest eines Katalysators in Verbindungen, die sich von Phenol abtrennen lassen, welches dadurch gekennzeichnet ist, daß die aus den Verunreinigungen durch Umwandlung mittels sauren Katalysators entstandenen Verbindungen vom Phenol thermisch abgetrennt werden und das Phenol zumindest ein weiteres Mal mit einem sauren Katalysator behandelt wird.

Ebenfalls ist Gegenstand der vorliegenden Erfindung ein Verfahren zur Herstellung von Phenol, wobei die Entfernung von Verunreinigungen aus Phenol durch Umwandeln dieser Verunreinigungen mittels zumindest eines Katalysators in Verbindungen, die sich von Phenol abtrennen lassen, erfolgt, welches dadurch gekennzeichnet ist, daß die aus den Verunreinigungen durch Umwandlung mittels sauren Katalysators entstandenen Verbindungen vom Phenol thermisch abgetrennt werden und das Phenol zumindest ein weiteres Mal mit einem sauren Katalysator behandelt wird.

Der Vorteil des erfindungsgemäßen Verfahrens besteht darin, daß bei der Entfernung von Verunreinigungen, welche die übliche Zusammensetzung aufweisen, aus Phenol, welches aus der säurekatalysierten Spaltung von Cumolhydroperoxid und anschließender destillativer Aufarbeitung des Spaltproduktes erhalten wird, keine Grenzwerte bezüglich eines bestimmten Inhaltstoffes der Verunreinigungen eingehalten werden müssen.

Im Gegensatz zu herkömmlichen Verfahren erfolgt die Entfernung von Verunreinigungen durch zweimaliges Behandeln des Phenols mit einem sauren Katalysator, wobei sich an die jeweilige Behandlung eine destillative Behandlung anschließt. Trotzdem das Verfahren einen Destillationsschritt mehr als die herkömmlichen Verfahren aufweist, kann der Energieaufwand durch Rückführung der eingesetzten Wärmeenergie gering gehalten werden.

Das erfindungsgemäße Verfahren wird im Folgenden beispielhaft beschrieben, ohne auf diese Ausführungsart beschränkt zu sein.

Mit Hilfe des erfindungsgemäßen Verfahrens zur Entfernung von Verunreinigungen aus Phenol mittels zumindest eines sauren Katalysators kann Phenol unterschiedlicher Herkunft gereinigt werden. Das Phenol kann bei Prozessen, die bei der Herstellung von Phenol durchgeführt werden, oder bei Prozessen, bei denen Phenol als Rohstoff eingesetzt wird, anfallen. Vorzugsweise handelt es sich bei dem Phenol um sogenanntes Rohphenol, welches bei der thermischen Aufarbeitung von Phenol enthaltenden Strömen anfällt. Ebenso kann das erfindungsgemäße Verfahren auf nicht ausreichend saubere Reinphenolströme angewendet werden. Ganz besonders bevorzugt stammt das Rohphenol aus der thermischen Aufarbeitung der Spaltproduktphase, welche bei der säurekatalysierten Spaltung von Cumolhydroperoxid anfällt.

Bei der thermischen Aufarbeitung von Phenol enthaltenden Phasen, welche üblicherweise durch Destillation oder Rektifikation erfolgt, werden Phenolfraktionen erhalten, die neben Phenol auch noch Verunreinigungen aufweisen, welche sich thermisch nicht oder nur unvollständig vom Phenol trennen lassen. Solche Verunreinigungen können z.B. Mesityloxid, Isomesityloxid, Methylisobuthylketon, Hydroxyaceton, Acetophenon, Methylcyclopentenon, Methylstyrol, Phenylketone und/oder Methylbenzofuran sein.

Erfindungsgemäß werden Phenolfraktionen, die Verunreinigungen aufweisendes Phenol enthalten, mit einem sauren Katalysator behandelt. Der saure Katalysator kann z.B. ein saures Ionenaustauscherharz oder ein supersaurer Katalysator sein. Als saures Ionenaustauscherharz kann jedes handelsübliche Ionenaustauscherharz verwendet werden. Solche Ionenaustauscherharze weisen z.B. aromatische Sulfonsäuregruppen vernetzt mit Polystyrol auf. Ein solches Ionenaustauscherharz, wie z.B. Amberlyst 15, ist bei der Firma Rohm und Haas erhältlich.

Als supersaure Katalysatoren können alle Katalysatoren verwendet werden, die eine Säurestärke aufweise die größer ist als die einer 100 %-igen Schwefelsäure. Solche Katalysatoren sind z.B. auf Salzen oder Oxiden immobilisiertes AlCl₃, SbF₅ oder Sulfatgruppen. Ähnliche Eigenschaften aufweisende und damit ebenfalls im erfindungsgemäßen Verfahren verwendbare Katalysatoren sind z.B. Heteropolysäuren aus Metalloxiden von Metallen wie z.B. Molybdän oder Wolfram.

Die Behandlung der Phenolfraktion erfolgt vorzugsweise an einem Ionenaustauscher in einem Festbett- oder Fließbettreaktor, vorzugsweise an einem Ionenaustauscher in einem Festbettreaktor. Die Behandlung der Phenolfraktion wird in Abhängigkeit von der thermischen Stabilität des Katalysators bei einer Temperatur von 100 bis 200 °C, bei der Verwendung von Ionenaustauschern bevorzugt bei einer Temperatur von 100 bis 130 °C durchgeführt. Nach der Behandlung, bei welcher sich ein Teil der Verunreinigungen durch verschiedene, säurekatalysierte Reaktionen in andere Verbindungen umgewandelt haben, werden diese, sowie bei diesen Reaktionen eventuell entstandenes Wasser von Phenol weitgehend getrennt. Das Trennen kann mechanisch und/oder thermisch erfolgen. Eine mechanische Trennung kann z.B. dann eingesetzt werden, wenn die als Verunreinigungen im Phenol vorhandenen Verbindungen mittels des sauren Katalysators in Verbindungen umgewandelt wurden, welche durch Phasentrennung, Filtration oder ähnliche Verfahren zum Trennen von in flüssig/flüssig, flüssig/fest oder flüssig/gasförmig Phasen vorliegenden Verbindungen, voneinander getrennt werden können. Thermische Trennmethoden sind z.B. die Flash-Verdampfung oder Destillation bzw. Rektifikation, aber auch fraktionierte Kristallisation bzw. Ausfrieren von Verbindungen. Vorzugsweise erfolgt das Trennen durch eine thermische Behandlung in einer Destillations- oder Rektifikationsapparatur, vorzugsweise in einer Destillationskolonne, wobei Verbindungen, die einen niedrigeren Siedepunkt als Phenol aufweisen, zum großen Teil über den Kopf der Kolonne abdestilliert werden und Verbindungen die einen höheren Siedepunkt als Phenol aufweisen als Sumpfprodukt in der Kolonne zurückbleiben. Das von den Verunreinigungen getrennte Phenol wird vorzugsweise über einen Seitenabzug aus der Kolonne entnommen.

Das aus dieser thermischen Behandlung erhaltene Phenol oder Phenol, welches mit einer anderen Trennmethode von Verbindungen, welche durch die Behandlung mit einem sauren Katalysator entstanden sind, abgetrennt wurde, wird auf eine Temperatur von weniger als 100 °C gebracht. Erfolgte die Trennung durch eine thermische Behandlung wird das Phenol auf eine Temperatur von weniger als 100 °C abgekühlt. Das Abkühlen des Phenols kann z.B. in einem Wärmetauscher erfolgen, in welchem das thermisch behandelte Phenol die Wärme an mit dem Katalysator noch zu behandelndes Phenol abgibt. Auf diese Weise läßt sich ein Teil der für die thermische Behandlung benötigte Wärmeenergie zurückgewinnen.

Das auf eine Temperatur von weniger als 100 °C gebrachte Phenol, welches von den bei der Behandlung mit einem sauren Katalysator aus den Verunreinigungen entstandenen Verbindungen sowie Reaktionswasser weitgehend getrennt wurde, wird erneut mit einem sauren Katalysator behandelt. Die Behandlung kann ebenfalls in einem Ionenaustauscher, einem Festbett- oder Fließbettreaktor durchgeführt werden. Vorzugsweise erfolgt die Behandlung ebenfalls wieder in einem Ionenaustauscher oder einem Festbettreaktor. Als saure Katalysatoren kann wie oben beschrieben wieder saures Ionenaustauscherharz und/oder ein supersaurer Katalysator verwendet werden. Diese zweite Behandlung des Phenols erfolgt vorzugsweise bei einer Temperatur von weniger als 100 °C, ganz besonders bevorzugt bei einer Temperatur von 75 bis 95 °C.

Das zum zweiten Mal mit zumindest einem sauren Katalysator behandelte Phenol wird wiederum einer mechanischen und/oder thermischen Trennung zugeführt, bei welcher wiederum Verbindungen, die aus den im Phenol vorhandenen Verunreinigungen durch Reaktion an zumindest einem sauren Katalysator entstanden sind, von dem Phenol abgetrennt werden. Die Trennung kann in Apparaturen wie oben beschrieben durchgeführt werden. Besonders bevorzugt wird die Trennung wiederum thermisch vorgenommen. Bei der Durchführung der thermischen Trennung werden wiederum Leichtsieder, Hochsieder und eventuell entstehende Reaktionswasser von dem Phenol getrennt. Durch die Trennung, insbesondere durch die thermische Trennung, erhält man eine Phenolfraktion, die weitestgehend von den Verunreinigungen befreit ist und einer weiteren Verarbeitung zugeführt werden kann.

Es kann vorteilhaft sein, wenn vor zumindest einer der erfindungsgemäßen mechanischen und/oder thermischen Trennungen der pH-Wert der zu behandelnden Phenolfraktion durch Zugabe einer Base angehoben wird. Vorzugsweise wird der pH-Wert des Phenols vor der thermischen Trennung durch Zugabe eines Alkalihydroxids und besonders bevorzugt durch Zugabe von Natriumhydroxid angehoben, um Rückspaltungen während der thermischen Trennung zu verhindern.

Es kann vorteilhaft sein, bei der Durchführung des erfindungsgemäßen Verfahrens die thermische Trennung in Destillationskolonnen durchzuführen, die es erlauben, einen Teil des Kopfproduktes und/oder einen Teil des Sumpfproduktes als Rücklauf in die Kolonne zurückzufahren.

Es kann vorteilhaft sein, erst mechanisch und dann thermisch zu trennen. Dies kann z.B. dann sinvoll sein, wenn sich zwischen Phenol und zumindest einem Teil der durch Umwandlung der Verunreinigungen an zumindest einem sauren Katalysator entstandenen Verbindungen eine Phasentrennung einstellt. In einem solchen Fall ist es vorteilhaft, erst die Phasen voneinander zu trennen, z.B. mit einem Phasenabscheider, und danach eine thermische Trennung vorzunehmen. Durch die Kombination von mechanischer und thermischer Trennung kann die für die thermische Trennung notwendige Energiemenge verringert werden, da ein kleineres Volumen bzw. eine kleinere Gesamtmenge erhitzt (Destillation) oder abgekühlt (Ausfrieren) werden muß.

Das erfindungsgemäße Verfahren kann diskontinuierlich oder kontinuierlich durchgeführt werden. Vorzugsweise wird das erfindungsgemäße Verfahren kontinuierlich durchgeführt.

Das erfindungsgemäße Verfahren kann auf alle Phenolströme, die Verunreinigungen aufweisen, angewendet werden. Insbesondere kann das erfindungsgemäße Verfahren zur Entfernung von Verunreinigungen aus Phenol mittels eines sauren Katalysators auf Phenolströme angewendet werden, die in Prozessen bei der Herstellung von Phenol nach dem Hock-Verfahren anfallen. Ganz besonders bevorzugt wird das erfindungsgemäße Verfahren auf Phenolströme angewendet, die bei der destillativen Aufarbeitung von Spaltprodukt, welches bei der säurekatalysierten Spaltung von Cumolhydroperoxid anfällt, erhalten werden.

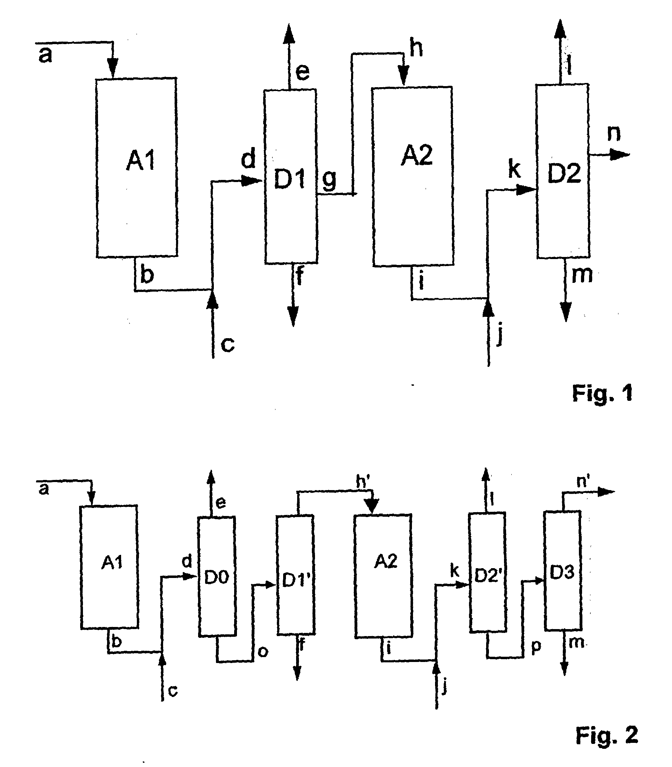

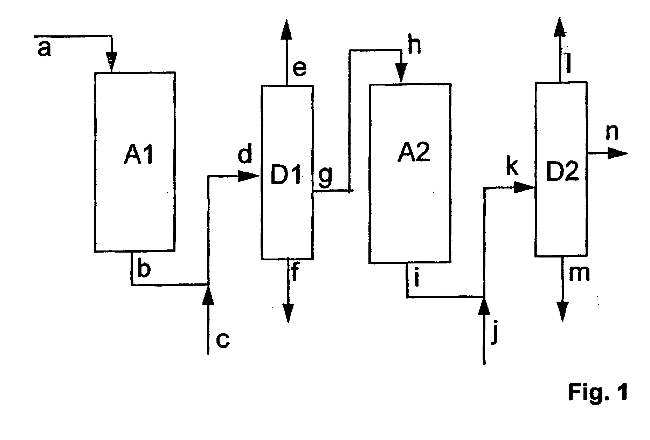

In Fig. 1 und Fig 2 sind mögliche Ausführungsarten des erfindungsgemäßen Verfahrens dargestellt, ohne daß das Verfahren auf diese beschränkt sein soll.

In Fig. 1 ist eine mögliche Ausführungsart des erfindungsgemäßen Verfahrens dargestellt. Bei dieser Ausführungsform wird über Leitung **a** das Verunreinigungen aufweisende Phenol in einen Ionenaustauscher **A1** geleitet, der einen sauren Katalysator aufweist. Über Leitung **b** wird das mit zumindest einem sauren Katalysator behandelte Phenol zur Destillationskolonne geleitet. Über Leitung **c** kann dem Phenol vor Eintritt in die Destillationskolonne **D1** über Leitung **d** eine Base zugemischt werden. Am Kopf der Destillationskolonne **D1** können Leichtsieder, die eine geringere Siedetemperatur als Phenol aufweisen, über Leitung **e** abgenommen werden. Aus dem Sumpf der Kolonne können über Leitung **f** Hochsieder, die eine Siedetemperatur, die über der von Phenol liegt, abgenommen werden. Über den Seitenabzug **g** gelangt die von Hoch- und Leichtsiedern weitgehend befreite Phenolfraktion über Leitung **h** in einen zweiten Ionenaustauscher **A2**. Die mit zumindest einem sauren Katalysator behandelte Phenolfraktion verläßt diesen Ionenaustauscher über Leitung **i**. Dieser Phenolfraktion kann über Leitung **j** wiederum eine Base zugemischt werden, bevor die Phenolfraktion über Leitung k in eine zweite Destillationskolonne geführt wird. Aus dieser Destillationskolonne **D2**, in welcher wiederum Phenol von Hoch- und Leichtsiedern getrennt wird, können über Kopf über Leitung **l** die Leichtsieder und über Leitung **m** aus dem Sumpf der Kolonne die Hochsieder entnommen und einer Aufarbeitung zugeführt werden. Aus dem Seitenabzug über Leitung **n** wird eine weitestgehend von Verunreinigungen befreite Phenolfraktion der Destillationskolonne entnommen und diese kann einer weiteren Aufarbeitung oder einer Verarbeitung zugeführt werden.

Fig. 2 zeigt eine alternative Ausführungsart des erfindungsgemäßen Verfahrens. Bei dieser Ausführungsform wird über Leitung **a** das Verunreinigungen aufweisende Phenol in einen Ionenaustauscher **A1** geleitet, der einen sauren Katalysator aufweist. Über Leitung **b** wird das mit zumindest einem sauren Katalysator behandelte Phenol zur Destillationskolonne geleitet. Über Leitung **c** kann dem Phenol vor Eintritt in die Destillationskolonne **D0** über Leitung **d** eine Base zugemischt werden. Am Kopf der Destillationskolonne **D0** können Leichtsieder, die eine geringere Siedetemperatur als Phenol aufweisen, über Leitung **e** abgenommen werden. Aus dem Sumpf der Kolonne kann über Leitung **o** ein Hochsieder und Phenol aufweisender Strom in die Destillationskolonne **D1'** geleitet werden. In dieser werden die Hochsieder vom Phenol abgetrennt. Über Leitung **f** werden diese Hochsieder, die eine Siedetemperatur aufweisen die über der von Phenol liegt, aus dem Sumpf der Kolonne abgenommen. Über Kopf wird die von Hoch- und Leichtsiedern weitgehend befreite Phenolfraktion über Leitung **h'** in einen zweiten Ionenaustauscher **A2** geleitet. Die mit zumindest einem sauren Katalysator behandelte Phenolfraktion verläßt diesen Ionenaustauscher über Leitung **i**. Dieser Phenolfraktion kann über Leitung **j** wiederum eine Base zugemischt werden, bevor die Phenolfraktion über Leitung **k** in eine weitere Destillationskolonne geführt wird. Aus dieser Destillationskolonne **D2'**, in welcher Leichtsieder abgetrennt werden, können über Kopf über Leitung **l** die Leichtsieder entnommen werden. Über Leitung wird **p** aus dem Sumpf der Kolonne ein Strom, der die Hochsieder und das Phenol aufweist, entnommen und seitlich in die Destillationskolonne **D3** eingespeist. Aus dieser Kolonne wird über Kopf über Leitung **n'** eine weitestgehend von Verunreinigungen befreite Phenolfraktion der Destillationskolonne entnommen und diese kann einer weiteren Aufarbeitung oder einer Verarbeitung zugeführt werden. Am Sumpf der Destillationskolonne **D3** kann über Leitung **m** eine Hochsieder, also Verbindungen mit einem Siedepunkt oberhalb dem von Phenol, abgenommen und einer Aufarbeitung zugeführt werden.

Das erfindungsgemäße Verfahren wird in dem folgenden Beispiel beschrieben, ohne daß das Verfahren auf diese beschränkt sein soll.

### Beispiel: Entfernung von Verunreinigungen aus Phenol

Ein Phenolstrom, der als Verunreinigungen unter anderem 300 wppm Mesityloxid, 3000 wppm Hydroxyaceton und 450 wppm Methylbenzofuran aufwies, wurde in einem Wärmetauscher auf eine Temperatur von 115 °C gebracht. Der Phenolstrom wurde über einen Festbettionenaustauscher geleitet, welcher mit dem Ionenaustauscherharz Amberlyst 15 befüllt war. Die Verweilzeit des Phenolstroms im Ionenaustauscher betrug 4 Stunden. Dem in diesem Ionenaustauscher behandelten Phenolstrom wurde soviel Natriumhydroxid zugesetzt, daß der pH-Wert des Phenolstroms ca. 7 betrug. Der so behandelte Phenolstrom wurde in eine Destillationskolonne überführt, in welcher der Phenolstrom thermisch behandelt wurde. Die Temperaturbereiche in der Destillationskolonne sind so gewählt worden, daß am Seitenabzug der Kolonne eine Phenolfraktion abgezogen werden konnte, während über Kopf überwiegend Verbindungen mit einer Siedetemperatur niedriger als der von Phenol und im Sumpf der Kolonne Verbindungen mit einer Siedetemperatur höher als der von Phenol abgenommen werden konnten. Durch eine Analyse der Phenolfraktion, welche aus dem Seitenabzug erhalten wurde, wurde der Gehalt an Mesityloxid mit < 10 wppm, an Hydroxyaceton mit < 30 wppm und an Methylbenzofuran mit < 1000 wppm ermittelt.

Diese Phenolfraktion wurde in einem Wärmetauscher auf eine Temperatur von 85 °C abgekühlt, wobei die abgegebene Wärme zum Aufheizen von thermisch zu behandelndem Phenol genutzt wurde. Die so vorbereitete Phenolfraktion wurde über einen Festbettionenaustauscher geleitet, der mit dem Ionenaustauscherharz Amberlyst 15 ausgerüstet war. Die Verweilzeit dieses Phenolstroms im Ionenaustauscher betrug 2,5 Stunden. Dieser mit einem sauren Katalysator behandelten Phenolfraktion wurde wiederum so viel Natriumhydroxid zugeführt, daß der pH-Wert der Phenolfraktion ca. 7 betrug. Die Phenolfraktion wurde über einen Wärmetauscher erwärmt und in eine weitere Destillationskolonne überführt. Wiederum wurde der Temperaturbereich in der Destillationskolonne so eingestellt, daß am Seitenabzug der Kolonne eine Phenolfraktion abgezogen werden konnte, während über Kopf überwiegend Verbindungen mit einer Siedetemperatur niedriger als der von Phenol und im Sumpf der Kolonne Verbindungen mit einer Siedetemperatur höher als der von Phenol abgenommen werden konnte. Durch eine Analyse der Phenolfraktion, welche aus dem Seitenabzug erhalten wurde, wurde der Gehalt an Mesityloxid mit < 1 wppm, an Hydroxyaceton mit < 1 wppm und an Methylbenzofuran mit < 10 wppm ermittelt. Anhand der Analyseergebnisse läßt sich sehr gut erkennen, daß die Gehalte an Verunreinigungen im Phenol durch die zweimalige Behandlung mit einem sauren Katalysator deutlich geringer sind, als bei den herkömmlichen Verfahren, bei denen die Behandlung mit einem sauren Katalysator nur einmal erfolgt ist.

## Patentansprüche

1. Verfahren zur Entfernung von Verunreinigungen aus Phenol durch Umwandeln dieser Verunreinigungen mittels zumindest eines Katalysators in Verbindungen, die sich von Phenol abtrennen lassen,
**dadurch gekennzeichnet,**
**daß** die aus den Verunreinigungen durch Umwandlung mittels sauren Katalysators entstandenen Verbindungen vom Phenol thermisch abgetrennt werden und das Phenol zumindest ein weiteres Mal mit einem sauren Katalysator behandelt wird.

2. Verfahren zur Herstellung von Phenol, wobei die Entfernung von Verunreinigungen aus Phenol durch Umwandeln dieser Verunreinigungen mittels zumindest eines Katalysators in Verbindungen, die sich von Phenol abtrennen lassen, erfolgt,
**dadurch gekennzeichnet,**
**daß** die aus den Verunreinigungen durch Umwandlung mittels sauren Katalysators entstandenen Verbindungen vom Phenol thermisch abgetrennt werden und das Phenol zumindest ein weiteres Mal mit einem sauren Katalysator behandelt wird.

3. Verfahren nach zumindest einem der Ansprüche 1 oder 2,
**dadurch gekennzeichnet,**
**daß** als saurer Katalysator ein supersaurer Katalysator und/oder ein saures Ionenaustauscherharz eingesetzt wird.

4. Verfahren nach zumindest einem der Ansprüche 1 bis 3,
**dadurch gekennzeichnet,**
**daß** die erste Behandlung des Phenols mit einem sauren Katalysator bei einer Temperatur von 100 bis 130 °C durchgeführt wird.

5. Verfahren nach zumindest einem der Ansprüche 1 bis 4,
**dadurch gekennzeichnet,**
**daß** eine zweite Behandlung des Phenols mit einem sauren Katalysator bei einer Temperatur von weniger als 100 °C durchgeführt wird.

6. Verfahren nach Anspruch 5,
**dadurch gekennzeichnet,**
**daß** das Phenol nach der zweiten Behandlung mit zumindest einem sauren Katalysator von den aus den Verunreinigungen durch Umwandlung mittels sauren Katalysators entstandenen Verbindungen getrennt wird.

7. Verfahren nach zumindest einem der Ansprüche 1 bis 6,
**dadurch gekennzeichnet,**
**daß** das Phenol nach einer Behandlung mit zumindest einem sauren Katalysator von den aus den Verunreinigungen durch Umwandlung mittels zumindest eines sauren Katalysators entstandenen Verbindungen mechanisch oder thermisch getrennt wird.

8. Verfahren nach Anspruch 7,
**dadurch gekennzeichnet,**
**daß** zur thermischen Trennung eine Rektifikation oder eine Destillation durchgeführt wird.

9. Verfahren nach zumindest einem der Ansprüche 1 bis 8,
**dadurch gekennzeichnet,**
**daß** dem Phenol, welches mit zumindest einem sauren Katalysator behandelt wurde, vor der thermischen Trennung eine Base zugefügt wird.

10. Verfahren nach Anspruch 9,
**dadurch gekennzeichnet,**
**daß** Phenol, welches mit zumindest einem sauren Katalysator behandelt wurde, vor der thermischen Trennung Natriumhydroxid als Base zugefügt wird.

11. Verfahren nach zumindest einem der Ansprüche 1 bis 10,
**dadurch gekennzeichnet,**
**daß** bei einer thermischen Trennung des mit zumindest einem sauren Katalysator behandelten Phenols Verbindungen mit einer niedrigeren Siedetemperatur und/oder Verbindungen mit einer höheren Siedetemperatur als der von Phenol vom Phenol abgetrennt werden.

12. Verfahren nach zumindest einem der Ansprüche 1 bis 11,
**dadurch gekennzeichnet,**
**daß** die in thermisch behandeltem und/oder mit zumindest einem sauren Katalysator behandeltem Phenol vorhandene Wärmeenergie auf destillativ zu behandelndes und/oder mit zumindest einem Katalysator zu behandelndes Phenol übertragen wird.

## Claims

1. A process of removing impurities from phenol by converting said impurities with at least one catalyst into compounds which can be separated from phenol,
**characterized in that**
the compounds obtained by conversion of the impurities with acid catalysts are thermally separated from phenol and **in that** the phenol is treated at least a second time with an acid catalyst.

2. A process for the synthesis of phenol wherein impurities are removed from phenol by conversion of said impurities with at least one catalyst into compounds which can be separated from phenol,
**characterized in that**
the compounds obtained by conversion of the impurities with acid catalysts are thermally separated from phenol and **in that** the phenol is treated at least a second time with an acid catalyst.

3. The process according to at least one of claims 1 or 2,
**characterized in that**
the acid catalyst is a superacid catalyst and/or an acid ion exchange resin.

4. The process according to at least one of claims 1-3,
**characterized in that**
the first treatment of the phenol with an acid catalyst is performed at a temperature of 100-130°C.

5. The process according to at least one of claims 1-4,
**characterized in that**
a second treatment of the phenol with an acid catalyst is performed at a temperature of less than 100°C.

6. The process according to claim 5,
**characterized in that**
after the second treatment with at least one acid catalyst the phenol is separated from the compounds obtained by conversion of the impurities with acid catalysts.

7. The process according to at least one of claims 1-6,
**characterized in that**
after treatment with at least one acid catalyst the phenol is separated mechanically or thermally from the compounds obtained by conversion of the impurities with at least one acid catalyst.

8. The process according to claim 7,
**characterized in that**
the thermal separation is performed by rectification or distillation.

9. The process according to at least one of claims 1-8,
**characterized in that**
before thermal separation a base is added to the phenol which has been treated with at least one acid catalyst.

10. The process according to claim 9,
**characterized in that**
before thermal separation sodium hydroxide is added as base to phenol which has been treated with at least one acid catalyst.

11. The process according to at least one of claims 1-10,
**characterized in that**
when thermally separating phenol which has been treated with at least one acid catalyst, compounds with a lower boiling temperature and/or compounds with a higher temperature than that of phenol are separated from phenol.

12. The process according to at least one of claims 1-11,
**characterized in that**
the thermal energy present in thermally treated phenol and/or in phenol treated with at least one acid catalyst is transferred to phenol which is to be treated by distillation and/or with at least one catalyst.

## Revendications

1. Procédé pour l'élimination d'impuretés du phénol par conversion de ces impuretés, au moyen d'au moins un catalyseur, en composés pouvant être séparés du phénol, **caractérisé en ce que** les composés formés à partir des impuretés au moyen d'un catalyseur acide sont séparés du phénol par voie thermique et que le phénol est traité au moins une fois encore par le catalyseur acide.

2. Procédé de préparation de phénol, où l'élimination d'impuretés du phénol est entreprise par conversion de ces impuretés, au moyen d'au moins un catalyseur, en composés pouvant être séparés du phénol, **caractérisé en ce que** les composés formés à partir des impuretés au moyen d'un catalyseur acide sont séparés du phénol par voie thermique et que le phénol est traité au moins une fois encore par un catalyseur acide.

3. Procédé selon au moins l'une des revendications 1 ou 2, **caractérisé en ce que** l'on met en oeuvre en tant que catalyseur acide un catalyseur superacide et / ou une résine échangeuse d'ions acide.

4. Procédé selon au moins l'une des revendications 1 à 3, **caractérisé en ce que** le premier traitement du phénol par un catalyseur acide est entrepris à une température de 100 à 130°C.

5. Procédé selon au moins l'une des revendications 1 à 4, **caractérisé en ce qu'**un deuxième traitement du phénol par un catalyseur acide est entrepris à une température inférieure à 100°C.

6. Procédé selon la revendication 5, **caractérisé en ce que** le phénol, après le deuxième traitement par au moins un catalyseur acide, est séparé des composés formés à partir des impuretés par conversion au moyen d'un catalyseur acide.

7. Procédé selon au moins l'une des revendications 1 à 6, **caractérisé en ce que** le phénol, après un traitement par au moins un catalyseur acide, est séparé des composés formés à partir des impuretés par conversion au moyen d'au moins un catalyseur acide par un procédé mécanique ou thermique.

8. Procédé selon la revendication 7, **caractérisé en ce que** l'on entreprend, pour la séparation thermique, une rectification ou une distillation.

9. Procédé selon au moins l'une des revendications 1 à 8, **caractérisé en ce que** le phénol, qui a été traité par au moins un catalyseur acide, est additionné d'une base avant la séparation thermique.

10. Procédé selon la revendication 9, **caractérisé en ce que** le phénol, qui a été traité par au moins un catalyseur acide, est additionné d'hydroxyde de sodium en tant que base avant la séparation thermique.

11. Procédé selon au moins l'une des revendications 1 à 10, **caractérisé en ce que**, lors d'une séparation thermique du phénol traité par au moins un catalyseur acide, on sépare du phénol des composés à température d'ébullition inférieure et / ou des composés à température d'ébullition supérieure à celle du phénol.

12. Procédé selon au moins l'une des revendications 1 à 11, **caractérisé en ce que** l'énergie thermique présente dans le phénol traité thermiquement et / ou traité par au moins un catalyseur acide est transférée au phénol à traiter par distillation et / ou à traiter par au moins un catalyseur.
